# EUROPEAN PATENT SPECIFICATION

(11) **EP 3 302 282 B1**
(45) Date of publication and mention of the grant of the patent: **14.04.2021**
(21) Application number: 16804265.3
(22) Date of filing: 31.05.2016
(51) Int. Cl.: A61B 6/03, A61B 6/00, H01J 35/30

(54) **SYSTEMS AND METHODS FOR REDUCING RADIATION DOSE IN CT**
SYSTEME UND VERFAHREN ZUR REDUZIERUNG DER STRAHLUNGSDOSIS BEI DER CT
SYSTÈMES ET PROCÉDÉS DE RÉDUCTION DE DOSE DE RAYONNEMENT EN TOMOGRAPHIE ASSISTÉE PAR ORDINATEUR

(30) Priority: 01.06.2015 US 201562169498 P
(43) Date of publication of application: 11.04.2018
(73) Proprietor: The Regents of the University of California, Oakland, CA 94607-5200 (US)
(72) Inventor: WANG, Danny JJ, Los Angeles, California 90095 (US); MARTIN, Thomas, Los Angeles, California 90095 (US); HOFFMAN, John M., Los Angeles, California 90024 (US)
(74) Representative: Richards, John
(86) International application number: PCT/US2016/035116
(87) International publication number: WO 2016/196521

(56) References cited:
- WO-A1-2014/085288
- WO-A1-2014/085288
- US-A1- 2006 008 048
- US-A1- 2006 115 050
- US-A1- 2006 115 050
- US-A1- 2007 092 058
- US-A1- 2008 056 432
- US-A1- 2008 056 432
- US-A1- 2008 144 765
- US-A1- 2010 038 973
- US-A1- 2010 038 973
- US-A1- 2010 046 712
- US-A1- 2010 104 062
- US-A1- 2010 135 455
- US-A1- 2010 215 141
- US-A1- 2012 057 671
- US-A1- 2013 272 504
- US-A1- 2013 272 504
- US-A1- 2015 098 548

## Description

### STATEMENT REGARDING FEDERALLY SPONSORED

### RESEARCH OR DEVELOPMENT

This invention was made with Government support under EB014922, awarded by the National Institutes of Health. The Government has certain rights in the invention.

### INCORPORATION-BY-REFERENCE OF COMPUTER PROGRAM APPENDIX

Not Applicable

### NOTICE OF MATERIAL SUBJECT TO COPYRIGHT PROTECTION

A portion of the material in this patent document is subject to copyright protection under the copyright laws of the United States and of other countries. The owner of the copyright rights has no objection to the facsimile reproduction by anyone of the patent document or the patent disclosure, as it appears in the United States Patent and Trademark Office publicly available file or records, but otherwise reserves all copyright rights whatsoever. The copyright owner does not hereby waive any of its rights to have this patent document maintained in secrecy, including without limitation its rights pursuant to 37 C.F.R. § 1.14.

### BACKGROUND

### 1. Technical Field

This description pertains generally to medical imaging, and more particularly to X-ray computed tomography (CT) systems and methods.

### 2. Background Discussion

X-ray computed tomography (CT) is commonly used in clinical practice. Compared to Magnetic Resonance Imaging (MRI), CT has the advantages of fast imaging speed and fewer contraindications, however radiation is a major concern for patient safety and long term health. In particular, dynamic CT scans such as CT perfusion (CTP) and CT angiography (CTA) involve high radiation dose due to the X-ray source remaining continuously on during the scan period (e.g. one minute). Over recent years, several adverse events of CT radiation overdose have been reported by media, and radiation dose has become a public health concern. The standard CT scan involves continuous rotation of the X-ray source around the patient. According to the Nyquist criterion, a total of π/2 * Xres (base resolution) projection views need to be acquired to form one CT image. For dynamic CT scans, the total number of X-ray projection views will be π/2 * Xres * Nframe (number of temporal frames, typically 45-60 for CT), resulting in a high level of radiation dose.

Accordingly, an object of the present disclosure is CT systems and methods to reduce radiation exposure

Document US2007092058 A1 may be considered to disclose a system for CT scanning of a subject, the system comprising: a pulse generator configured to be coupled to an X-ray source of a CT scanner, the X-ray source being mounted on gantry so as to rotate on cylindrical enclosure of the CT scanner; the pulse generator configured to periodically switch off emission of X-rays from the X-ray source; and application software coupled to the pulse generator; the application software comprising instructions to control timing of the pulse generator so as to intermittently expose a subject to X-rays from the X-ray source at pre-specified rotation angles of the gantry; application software coupled to an output of the CT scanner for receiving pulsed images from the CT scanner, the pulsed images corresponding to exposures at said pre-specified rotation angles; the application software comprising instructions for reconstructing each of said exposures to generate a reconstructed image.

### BRIEF SUMMARY

An aspect of the present description is low-dose CT imaging system and method that operates according to a pulsed X-ray emission scheme according to a predefined sequence of rotation angles of the X-ray source, along with image reconstruction algorithms to achieve high spatial and temporal resolution for CT scans. The systems and methods involve high speed switching (one the order of milliseconds) to generate pulsed exposure of X-ray radiation to the patient, reducing radiation dose by 4-8 folds or more compared to standard CT scans, without degrading image quality. The systems and methods of the present description allow for body CT perfusion scans that were previously not feasible due to the high radiation dose.

In one embodiment, the system of the present description may include hardware and software components, wherein the hardware allows a user to adjust dose reduction via number of projections acquired and obtain projections at predefined sequences of angles that are optimized for the reconstruction software. Novel projection view sharing techniques may be implemented, as well as iterative and/or constrained reconstruction algorithms. Three representative sequences for rotation angles may include, but are not limited to 1) angle-bisect (or bit-reverse); 2) golden-ratio; and 3) pseudo-random schemes.

Further aspects of the technology will be brought out in the following portions of the specification, wherein the detailed description is for the purpose of fully disclosing preferred embodiments of the technology without placing limitations thereon.

### BRIEF DESCRIPTION OF THE SEVERAL VIEWS

### OF THE DRAWING(S)

The technology described herein will be more fully understood by reference to the following drawings which are for illustrative purposes only:
FIG. 1 shows a schematic diagram of a low-dose CT scanning system according to an embodiment of the technology of this disclosure.
FIG. 2 shows a high-level block diagram of hardware and software components used for the low-dose CT scanning system.
FIG. 3 shows an angle-bisect or bit-reverse scheme with 4 rotations (A, B, C, D) according to a CT exposure scheme of the present description.
FIG. 4A through FIG. 4C show various projections orders in accordance with scanning methods used in the system of the present description: FIG. 4A, standard projection order based on a fixed increment (P=10); FIG. 4B, projection order based on the Golden angle increment of 111.25°; and FIG. 4C, projection order based on the Tiny Golden angle increment of 23.62°.
FIG. 5A shows a schematic diagram of an exemplary pulsed X-ray CT tube in a closed, non-illuminating mode in accordance with an embodiment the present description.
FIG. 5B shows a schematic diagram of an exemplary pulsed X-ray CT tube in an open, illuminating mode in accordance with an embodiment the present description.
FIG. 6 shows a detailed diagram of the KWIC image reconstruction for the golden angle projection method of FIG. 4B.
FIG. 7A through FIG. 7D show dynamic, simulated CT phantoms of a 5mm object (white dot) using FBP with varying projections.
FIG. 7E through FIG. 7H show dynamic, simulated CT phantoms a 5mm object (white dot) using KWIC with varying projections.
FIG. 8 shows a graph of clinical CT perfusion (CTP) signal curves of contrast uptake
FIG. 9A and FIG. 9B show relative CBV (rCBV) maps reconstructed using FBP and KWIC, respectively. The temporal fidelity is preserved with up to 25% dose reduction using KWIC.

### DETAILED DESCRIPTION

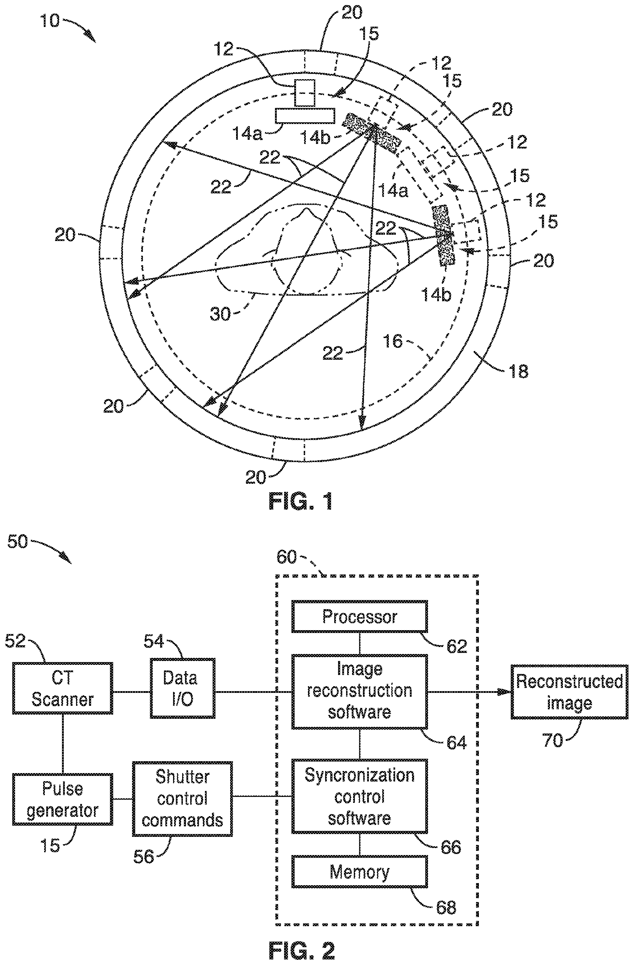

FIG. 1 shows a side-view schematic diagram of a low-dose CT scanning system 10 according to the present disclosure. Low-dose CT scanning system 10 reduces the radiation dose of CT scans by controlling the X-ray source to be on intermittently (instead of continuously) at pre-specified rotation angles. The dynamic CT image series can then be reconstructed using algorithms that preserve high spatial and temporal resolutions as well as image quality comparable to those of standard scans.

The CT scanning system 10 comprises a cylindrical enclosure 18 with a plurality of detectors 20 disposed circumferentially in a stationary ring along the inner wall of the X-ray enclosure 18. While a handful of detectors 20 are shown in the side view of FIG. 1, it is appreciated that any number of detectors 20 may be used incrementally along the circumference of the ring. Additionally, a number of rings or rows may be disposed axially down the tube 18.

An X-ray source 12 is disposed in the enclosure 18 on a gantry 16 that rotates around the circumference of the inner wall of tube 18. FIG. 1 shows the X-ray source 12 in four orientations (e.g. starting from 12 o'clock to 3 o'clock) in FIG. 1. An X-ray pulse generator 15 is integrated with or attached to the X-ray source 12 to control the emission of X-rays from the source into the enclosure 18. Pulse generator 15 is shown in FIG. 1 as being disposed in front of X-ray source 12. However, it is appreciated that the pulse generator 15 may be implemented within X-ray source 12, as will be explained in further detail below.

As seen in FIG. 1, a simplistic emission scenario is depicted wherein the pulse generator 15 is alternating between an off-state at 14a and an on-state at 14b. In the off-state 14a, no X-rays are emitted from the source 12. In the on state-14b, the pulse generator 15 allows X-rays 22 to be emitted into the enclosure 18, passing through the patient 30 for detection by one or more detectors 20 on the opposite wall of the enclosure 18.

The pulse generator 15 may comprise different configurations having distinct principles of operation. In one embodiment, the pulse generator 15 comprises a mechanical shutter or lead shield that acts as blinds or a shutter that opens and closes at high speed (on the order of milliseconds) while the source 12 is continuously powered. The shutter is configured to restrict emission of X-rays 22 in the off-state 14a, and opens up to allow emission X-rays 22 in the on-state 14b.

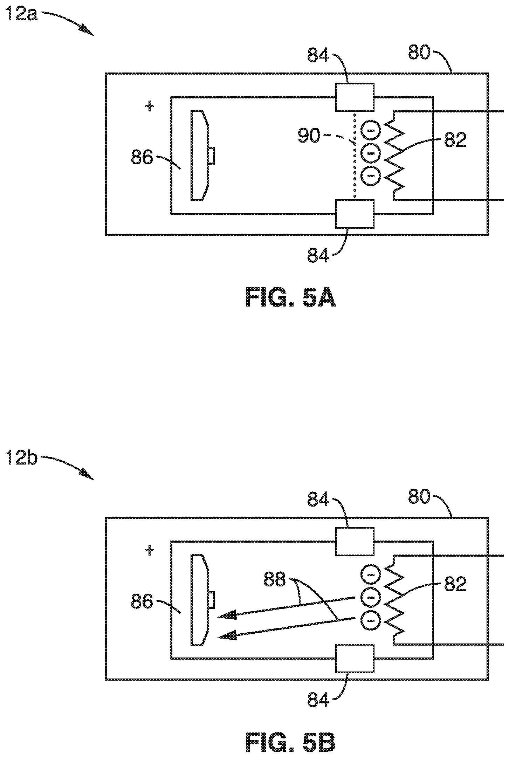

In an alternative embodiment, the pulse generator 15 operates via electromagnetic means using the modified X-ray source 80 shown in FIG. 5A and FIG. 5B, described in further detail below.

FIG. 2 shows a high-level block diagram of hardware and software components used for the low-dose CT scanning system 50. From the hardware perspective, an existing CT scanner 52 is equipped with a pulse generator 15 for modulating the X-ray emission from the X-ray source 12 within in the scanner. It is appreciated that scanner 52 may be integrated with the pulse generator 15, either with a mechanical shutter built in to the source 12, or via electromagnetically pulsed operation via a modified CT X-ray source 12a/12b shown in FIG. 5A and FIG. 5B.

On the software end, computer or server 60 may comprise image reconstruction software 64, synchronization control software 66 stored in memory 68 and operable on processor 62. Synchronization control software 66 contains instructions for operating pulse generator 15, in the form of shutter control commands 56 that control the timing of the pulsing of the X-rays, as will be described in further detail below. Image reconstruction software 64 comprises instructions for taking the output data 54 from the CT scanner 52 and reconstructing the data detected from the pulsed X-ray emission to generate a reconstructed image 70.

Synchronization control software 66 is configured to control sequencing of the pulsing as the gantry 16 rotates the X-ray source 12 within the enclosure 18.

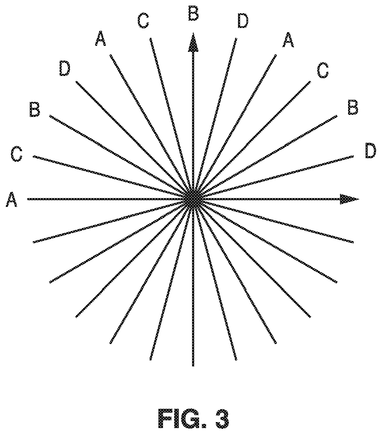

In one embodiment illustrated in FIG. 3, synchronization control software 66 uses angle-bisect or bit-reverse sequence for the rotation angle at which X-ray exposure occurs. In the angle-bisect or bit-reverse scheme the full projection angles are acquired in an interleaved fashion (A, B, C, D). During the first gantry rotation, only one set of evenly distributed projection angles are acquired at position A (e.g. at 60° intervals). During subsequent gantry 16 rotations, projections that bisect the previous set of projections are acquired (position B intersects previous A positions, position C intersects A to B positions, and position D then intersects B to A positions) until the full projection angles are reached.

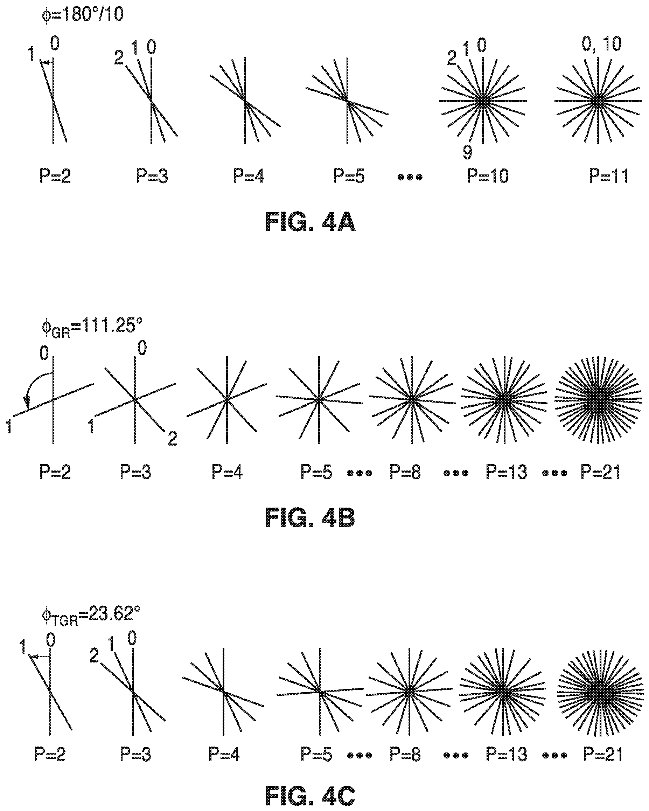

FIG. 4A through FIG. 4C show various projections orders in accordance with other scanning methods that may be implemented in the control software 66 of the system 50. FIG. 4A shows a standard projection order based on a fixed increment (P=10), or 18° projection increments.

FIG. 4B shows a projection order based on the Golden angle increment of 111.25°. In this configuration, the rotation angles of the X-ray source 12 are spaced by the golden angle (180°/1.618=111.25°) which guarantees an optimal projection distribution for any arbitrary number of projections used in reconstruction.

FIG. 4C shows a projection order based on the Tiny Golden angle increment of 23.62°. In this configuration, the rotation angles of the X-ray source 12 are spaced by the angle 23.62°, which guarantees an optimal projection distribution with the number of projections is greater than 7 for the shown angle increment.

Pseudo-random schemes may also be implemented, which are optimized for modern sparse sampling techniques with constrained reconstruction, such as compressed sensing.

Referring to FIG. 5A and FIG. 5B, high-speed power switching of the X-ray source 12 may be realized with a pulse generator 15 that operates via deflection of the electron beam off the tube anode using a magnetic field.

FIG. 5A shows a schematic diagram of an exemplary pulsed X-ray CT tube 80 in a closed, non-illuminating mode 12a. In this configuration, the griddling electrode 84 is configured to have a high enough negative potential so as to prevent electron flow from the cathode 82, essentially forming an electromagnetic field-based shield or barrier 90 between cathode 82 and anode 86. Production of X-rays is stopped, allowing for pulsed distribution of X-rays into the enclosure 18.

FIG. 5B shows a schematic diagram of the pulsed X-ray CT tube 80 in an open, illuminating mode 12b. In this mode, the griddling electrode 84 potential is modified to focus the electron beam 88 on to the anode 86, resulting in generation of the X-rays from the source 12b.

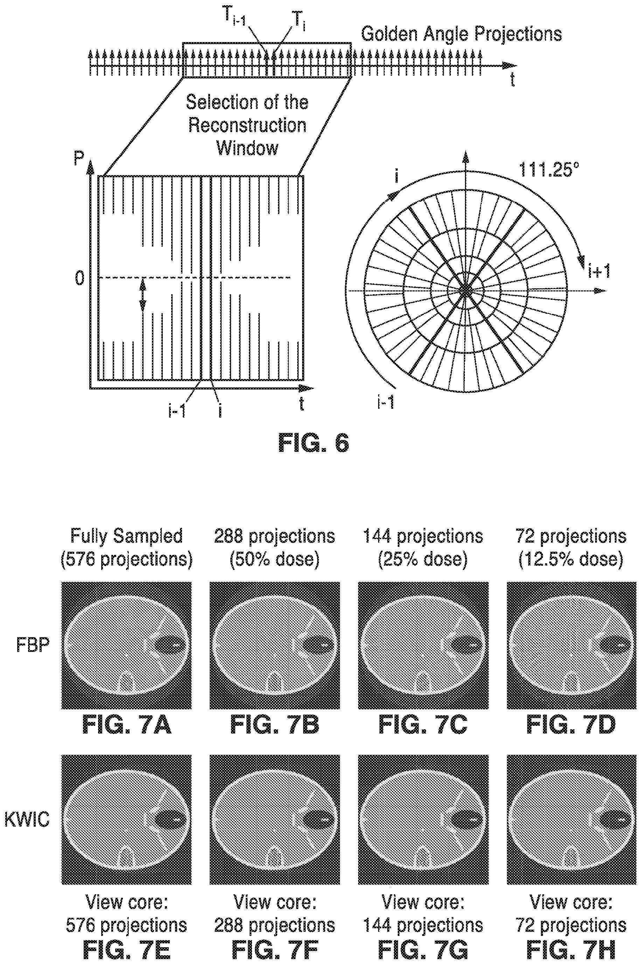

To reconstruct the full set of dynamic CT images 70, image reconstruction software 64 incorporates projection view sharing techniques of K-space Weighted Image Contrast (KWIC). KWIC may be implemented for any of the angle-bisect scheme (FIG. 4A) Golden angle scheme (FIG. 4B) or Tiny Golden angle scheme (FIG. 4C) for projection acquisition rotation angles of the X-ray source. FIG. 6 shows a diagram of KWIC reconstruction of dynamic CT scanning with golden angle projections. Using KWIC, the central 2DFT space (similar to k-space in MRI), which determines the image contrast, is sampled by the projection views of the time frame of interest (Tᵢ₋₁, Tᵢ in FIG. 6), whereas the peripheral 2DFT space is filled by projection views of neighboring time frames (similar to view sharing). Therefore, both high spatial and temporal resolutions can be achieved for dynamic CT scans using KWIC for any of the Golden ratio, Tiny Golden ration, and Bit-reverse schemes, as KWIC preserves undersampled CT image quality by proportionately increasing the number of encoded projections for more distant regions of the 2D Fourier Transform (FT) space. The image reconstruction software 64 employing KWIC is able to achieve a 10 fold reduction of radial projection views compared to standard techniques, which can be translated to 10 fold reduction of radiation dose for dynamic CT scans.

Table 1 shows an example software code for implementing the CT KWIC reconstruction algorithm, which provides an exemplary configuration of instructions that may be used for image reconstruction software 64.

The aforementioned KWIC reconstruction techniques were applied on a FORBILD CT head phantom as well as a clinical CT perfusion data set, resulting in a simulated 4-8x dose reduction while preserving the image quality and quantification accuracy for perfusion parameters.

FIG. 7A through FIG. 7D show dynamic, simulated CT phantoms of a 5mm object (white dot) using standard filtered back projection (FBP) construction with full radiation dose with varying amounts of projections. FIG. 7E through FIG. 7H show dynamic, simulated CT phantoms of the same 5mm object (white dot) using the CT-KWIC reconstruction with the same varying amounts of projections. The total number of projections per gantry rotation was reduced to obtain down to 12.5% of the original dose. The KWIC reconstruction algorithm preserves image quality that is lost to sampling artifacts in FBP.

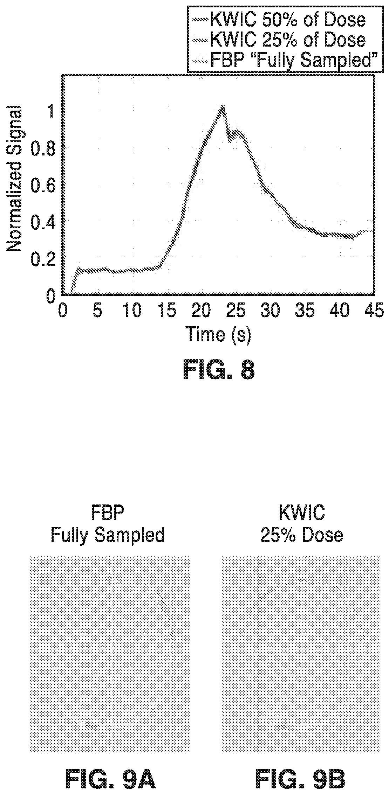

It is contemplated that even higher dose savings may be possible with refinements to the CT-KWIC reconstruction algorithm used for this demonstration. FIG. 8 shows a graph of clinical CT perfusion (CTP) signal curves of contrast uptake for KWIC at 50% dose, 25% dose, and fully sampled FBP.

FIG. 9A and FIG. 9B show relative CBV (rCBV) maps reconstructed using FBP and KWIC, respectively. The temporal fidelity is preserved with up to 25% dose reduction using KWIC.

The systems and methods of the present description are shown in a preferred configuration directed to dynamic CT. However, it is appreciated that systems and methods of the present description may be configured for implementation with other CT imaging modalities.

The low-dose dynamic CT systems and methods described herein provide for precise CT imaging with substantially reduced dose to patients undergoing CT perfusion and angiographic exams. The low-dose dynamic CT systems and methods may be configured to allow patients to have multiple low dose CTP and CTA exams for more frequent and regular monitoring of their diseases, which could improve patient outcome. Such dose reductions may also allow for body perfusion (e.g. in the liver or kidneys) where it has previously been too high dose to be a viable diagnostic or study option.

Embodiments of the present technology may be described with reference to flowchart illustrations of methods and systems according to embodiments of the technology, and/or algorithms, formulae, or other computational depictions, which may also be implemented as computer program products. In this regard, each block or step of a flowchart, and combinations of blocks (and/or steps) in a flowchart, algorithm, formula, or computational depiction can be implemented by various means, such as hardware, firmware, and/or software including one or more computer program instructions embodied in computer-readable program code logic. As will be appreciated, any such computer program instructions may be loaded onto a computer, including without limitation a general purpose computer or special purpose computer, or other programmable processing apparatus to produce a machine, such that the computer program instructions which execute on the computer or other programmable processing apparatus create means for implementing the functions specified in the block(s) of the flowchart(s).

Accordingly, blocks of the flowcharts, algorithms, formulae, or computational depictions support combinations of means for performing the specified functions, combinations of steps for performing the specified functions, and computer program instructions, such as embodied in computer-readable program code logic means, for performing the specified functions. It will also be understood that each block of the flowchart illustrations, algorithms, formulae, or computational depictions and combinations thereof described herein, can be implemented by special purpose hardware-based computer systems which perform the specified functions or steps, or combinations of special purpose hardware and computer-readable program code logic means.

Furthermore, these computer program instructions, such as embodied in computer-readable program code logic, may also be stored in a computer-readable memory that can direct a computer or other programmable processing apparatus to function in a particular manner, such that the instructions stored in the computer-readable memory produce an article of manufacture including instruction means which implement the function specified in the block(s) of the flowchart(s). The computer program instructions may also be loaded onto a computer or other programmable processing apparatus to cause a series of operational steps to be performed on the computer or other programmable processing apparatus to produce a computer-implemented process such that the instructions which execute on the computer or other programmable processing apparatus provide steps for implementing the functions specified in the block(s) of the flowchart(s), algorithm(s), formula(e), or computational depiction(s).

It will further be appreciated that the terms "programming" or "program executable" as used herein refer to one or more instructions that can be executed by a processor to perform a function as described herein. The instructions can be embodied in software, in firmware, or in a combination of software and firmware. The instructions can be stored local to the device in non-transitory media, or can be stored remotely such as on a server, or all or a portion of the instructions can be stored locally and remotely. Instructions stored remotely can be downloaded (pushed) to the device by user initiation, or automatically based on one or more factors. It will further be appreciated that as used herein, that the terms processor, computer processor, central processing unit (CPU), and computer are used synonymously to denote a device capable of executing the instructions and communicating with input/output interfaces and/or peripheral devices.

From the description herein, it will be appreciated that that the present disclosure encompasses multiple embodiments which include, but are not limited to, the following:
1. A system for low dose CT scanning of a subject, the system comprising: (a) a pulse generator configured to be coupled to an X-ray source of a CT scanner, the X-ray source being mounted on gantry so as to rotate within a cylindrical enclosure of the CT scanner; the pulse generator configured to periodically switch off emission of X-rays from the X-ray source into the cylindrical enclosure; and (b) application software coupled to the pulse generator; the application software comprising instructions to control timing of the pulse generator so as to intermittently expose a subject to X-rays from the X-ray source at pre-specified rotation angles of the gantry.
2. The system of any preceding embodiment: wherein the pulse generator comprises a mechanical shutter coupled to the x-ray source; wherein the shutter comprises an off-state to restrict X-rays from being emitted from the X-ray source and an on-state configured to allow X-rays to be emitted from the X-ray source into the enclosure; wherein the shutter is coupled to the application software to receive said instructions; and wherein the instructions comprise commands for timing the on-state of the shutter and resulting X-ray exposure at said pre-specified rotation angles of the gantry.
3. The system of any preceding embodiment: wherein the pulse generator comprises an off-state to restrict X-rays from being emitted from the X-ray pulse generator source and an on-state configured to allow X-rays to be emitted from the X-ray source into the enclosure; and wherein the pulse generator is coupled to the X-ray source to electromagnetic shield the X-ray source from emitting X-rays in the off-state; wherein the instructions comprise commands for timing the on-state of the pulse generator and resulting X-ray exposure at said pre-specified rotation angles of the gantry.
4. The system of any preceding embodiment: wherein the X-ray source comprises an anode, a cathode and a griddling electrode there between; and wherein the pulse generator is configured to modify a negative potential of the griddling electrode to form an electromagnetic field-based shield so as to prevent electron flow from the cathode the anode, thereby stopping emission of X-rays in the off-state.
5. The system of any preceding embodiment, wherein said pre-specified angles of rotation comprise a sequence of rotation angles selected from the group of rotation schemes consisting of: an angle-bisect scheme; a Golden-ratio scheme; or a Tiny Golden-ratio scheme.
6. The system of any preceding embodiment, further comprising: application software coupled to an output of the CT scanner for receiving pulsed images from the CT scanner, the pulsed images corresponding to exposures at said pre-specified rotation angles; the application software further configured for reconstructing each of said exposures to generate a reconstructed image.
7. The system of any preceding embodiment, wherein the reconstructed image is generated via a projection view sharing techniques.
8. The system of any preceding embodiment, wherein K-space Weighted Image Contrast (KWIC) is used to generate the reconstructed image.
9. An apparatus for lowering X-ray dose to a subject in a CT scanner, the CT scanner comprising an X-ray source being mounted on gantry so as to rotate within a cylindrical enclosure of the CT scanner, and a pulse generator coupled to the X-ray source to periodically switch off emission of X-rays from the X-ray source into the cylindrical enclosure, the apparatus comprising: (a) a computer processor coupled to the CT scanner; and (b) a non-transitory computer-readable memory storing instructions executable by the computer processor; (c) wherein said instructions, when executed by the computer processor, perform steps comprising: (i) controlling timing of the pulse generator so as to intermittently expose a subject to X-rays from the X-ray source at pre-specified rotation angles of the gantry; (ii) receiving pulsed images from the CT scanner, the pulsed images corresponding to exposures at said pre-specified rotation angles; and (iii) reconstructing each of said exposures to generate a reconstructed image.
10. The apparatus of any preceding embodiment: wherein the pulse generator comprises a mechanical shutter coupled to the x-ray source; wherein the shutter comprises an off-state to restrict X-rays from being emitted from the X-ray source and an on-state configured to allow X-rays to be emitted from the X-ray source into the enclosure; wherein the shutter is coupled to the application software to receive said instructions; and wherein the instructions comprise commands for timing the on-state of the shutter and resulting X-ray exposure at said pre-specified rotation angles of the gantry.
11. The apparatus of any preceding embodiment: wherein the pulse generator comprises an off-state to restrict X-rays from being emitted from the X-ray pulse generator source and an on-state configured to allow X-rays to be emitted from the X-ray source into the enclosure; wherein the pulse generator is coupled to the X-ray source to electromagnetic shield the X-ray source from emitting X-rays in the off-state; and wherein the instructions comprise commands for timing the on-state of the pulse generator and resulting X-ray exposure at said pre-specified rotation angles of the gantry.
12. The apparatus of any preceding embodiment: wherein the X-ray source comprises an anode, a cathode and a griddling electrode there between; and wherein the instructions are configured to modify a negative potential of the griddling electrode to form an electromagnetic field-based shield so as to prevent electron flow from the cathode the anode, thereby stopping emission of X-rays in the off-state.
13. The apparatus of any preceding embodiment, wherein said pre-specified angles of rotation comprise a sequence of rotation angles selected from the group of rotation schemes consisting of: an angle-bisect scheme; a Golden-ratio scheme; or a Tiny Golden-ratio scheme.
14. The apparatus of any preceding embodiment, wherein the reconstructed image is generated via a projection view sharing techniques.
15. The apparatus of any preceding embodiment, wherein K-space Weighted Image Contrast (KWIC) is used to generate the reconstructed image.
16. A low dose CT scanner for generating CT images of a subject, the CT scanner comprising: (a) an X-ray source disposed within a cylindrical enclosure; the cylindrical enclosure comprising a plurality of detectors configured to detect X-rays emitted from the X-ray source; the X-ray source mounted on a gantry so as to rotate within the cylindrical enclosure of the CT scanner; (b) a pulse generator coupled to the X-ray source; the pulse generator configured to periodically switch off emission of X-rays from the X-ray source into the cylindrical enclosure; and (c) application software coupled to the pulse generator; the application software comprising instructions to control timing of the pulse generator so as to intermittently expose a subject to X-rays from the X-ray source at pre-specified rotation angles of the gantry.
17. The CT scanner of any preceding embodiment: wherein the pulse generator comprises a mechanical shutter coupled to the x-ray source; wherein the shutter comprises an off-state to restrict X-rays from being emitted from the X-ray source and an on-state configured to allow X-rays to be emitted from the X-ray source into the enclosure; wherein the shutter is coupled to the application software to receive said instructions; and wherein the instructions comprise commands for timing the on-state of the shutter and resulting X-ray exposure at said pre-specified rotation angles of the gantry.
18. The CT scanner of any preceding embodiment: wherein the pulse generator comprises an off-state to restrict X-rays from being emitted from the X-ray pulse generator source and an on-state configured to allow X-rays to be emitted from the X-ray source into the enclosure; wherein the pulse generator is coupled to the X-ray source to electromagnetic shield the X-ray source from emitting X-rays in the off-state; and wherein the instructions comprise commands for timing the on-state of the pulse generator and resulting X-ray exposure at said pre-specified rotation angles of the gantry
19. The CT scanner of any preceding embodiment: wherein the X-ray source comprises an anode, a cathode and a griddling electrode there between; and wherein the pulse generator is configured to modify a negative potential of the griddling electrode to form an electromagnetic field-based shield so as to prevent electron flow from the cathode the anode, thereby stopping emission of X-rays in the off-state.
20. The CT scanner of any preceding embodiment, wherein said pre-specified angles of rotation comprise a sequence of rotation angles selected from the group of rotation schemes consisting of: an angle-bisect scheme; a Golden-ratio scheme; or a Tiny Golden-ratio scheme.
21. The CT scanner of any preceding embodiment, further comprising: application software coupled to the plurality of detectors for receiving pulsed images corresponding to exposures at said pre-specified rotation angles; the application software further configured for reconstructing each of said exposures to generate a reconstructed image.
22. The CT scanner of claim 21, wherein the reconstructed image is generated via a projection view sharing techniques.
23. The CT scanner of claim 22, wherein K-space Weighted Image Contrast (KWIC) is used to generate the reconstructed image.
24. A method for lowering X-ray dose to a subject in a CT scanner, the CT scanner comprising an X-ray source mounted on gantry so as to rotate within a cylindrical enclosure of the CT scanner and emit of X-rays into the cylindrical enclosure, the method comprising: (a) intermittently exposing a subject within the enclosure to X-rays from the X-ray source at pre-specified rotation angles of the gantry; (b) receiving pulsed images from the CT scanner, the pulsed images corresponding to exposures at said pre-specified rotation angles; and (c) reconstructing each of said exposures to generate a reconstructed image.
25. The method of any preceding embodiment: wherein the CT scanner comprises a mechanical shutter coupled to the x-ray source; wherein the shutter comprises an off-state to restrict X-rays from being emitted from the X-ray source and an on-state configured to allow X-rays to be emitted from the X-ray source into the enclosure; and wherein intermittently exposing a subject comprises timing the on-state of the shutter and resulting X-ray exposure at said pre-specified rotation angles of the gantry.
26. The method of any preceding embodiment: wherein the X-ray source comprises an electron beam being focused on an anode from a cathode to generate said X-rays; and wherein intermittently exposing a subject comprises deflecting the electron beam off the anode using a magnetic field, thereby restricting emission of X-rays from the X-ray source into the enclosure to control X-ray exposure to the subject only at said pre-specified rotation angles of the gantry.
27. The method of any preceding embodiment: wherein the X-ray source further comprises a griddling electrode between the anode and the cathode; and wherein deflecting the electron beam off the anode comprises generating sufficient negative potential within the griddling electrode to form an electromagnetic field-based shield so as to prevent electron flow from the cathode the anode, thereby stopping emission of X-rays for pre-specified rotation angles.
28. The method of any preceding embodiment, wherein said pre-specified angles of rotation comprise a sequence of rotation angles selected from the group of rotation schemes consisting of: an angle-bisect scheme; a Golden-ratio scheme; or a Tiny Golden-ratio scheme.
29. The method of any preceding embodiment, wherein the reconstructed image is generated via a projection view sharing techniques.
30. The method of any preceding embodiment, wherein K-space Weighted Image Contrast (KWIC) is used to generate the reconstructed image.

Although the description herein contains many details, these should not be construed as limiting the scope of the disclosure but as merely providing illustrations of some of the presently preferred embodiments.

**Table 1**

| |
|---|
| ```
 % CT KWIC Reconstruction algorithm based on MRI KWIC Recon
 % This file reconstructs a CT dynamic sinogram data set
 %%%%%%%%%%%%%%%%%%%%%%%%%%%%%%%%%%%%
 %%%%%%%%%
 %%%%%%%%%%%%%%%%%%%%%%%%%%%%%%%%%%%%%%%%%
 %%%%%%%%%
 %%%%%%%%%%%%%%%%%%%%% Variables for Recon
 %%%%%%%%%%%%%%%%%%%%%%%%%%%
 %%%%%%%%%
 %%%%%%%%%%%%%%%%%%%%%%%%%%%%%%%%%%%%%%%%%%%%
 %%%%%%%%%
 clear; clc; % close all;
 %%%%%%%%%%%%% Parameters used for KWIC Simulation
 view_core= [580]; % Number of radial views or projections in the
 center ring
 ringNum= [1]; %Number of rings for the KWIC reconstruction
 Nd = 672; % Number of detectors/resolution
 Nproj = 1160; %number of projections per gantry rotation
 nyq = [1]; % choose a ratio. nyq = 1 is the optimized kspace just
 satisfying the nyquist criteria, nyq < 1 over sampling, nyq >
 undersampling
 nyq_str = [1] ;
 time_length = 44; %sec
 data1 = 'ct_kwic_clinical.mat';
 angle_first = 1; %this is the index where the starting tube angle
 array is located or desired
 load(data1)
 tube_angles = tube_angles*pi/180;
 t_1 = (0:time_length/(Nproj*time_length):time_length-
 time_length/(Nproj*time_length));
``` |
| ```
 th_1 = repmat(tube_angles(1:Nproj),[1 time_length]);
 sino_stack_rcn_1 = sino_stack_ds(1:Nproj/2,:,1:time_length);
 sino_Angles_2 = th_1(1:Nproj/2,:); % in radians, starts at 0
 for vc = 1:length(view_core)
 for ny = 1:length(nyq)
     t1 = reshape(t_1,Nproj,time_length);
     % th_3 = reshape(th_1,Nproj,time_length);
     sino_stack_4 = sino_stack_rcn_1;
     time = t1(1:Nproj/2,:);
     % sino_stack = s(1:Nproj/2,:);
     % sino_angles = t-t(1); %in radians, starts at 0
     % sino_Angles_2 = sino_angles(1:Nproj/2)';
     %%%%%%%%%%%%% CT Phantom
     %%%%%%%%%%%%% Parameters
     t_total = time_length-1; %seconds, t_total/t_step + 1
     should be a multiple of the number of subapertures for convenience
     t_step = 1; %seconds
     FOV = 50; %cm Field of Fiew
     std_n = 0; %standard deviation of noise (0.01)
     noise = 2; %1 for Recian noise and anything else for
     Poisson noise
     n_proj_turn_max = Nproj; %maximum number of projections
     per turn (360 degrees)
     Precision = 0.0001; %This is to be able to match the right
     angles with the sinogram
``` |
| ```
     %%%%%%%%%%%% Parameters for gridding
     nLine_ext=1;
     flag_weighted=1; %Used for regridding/density compensation
     flag_kwic=1;%Use for regridding/density compensation
     %%%%%%%%%%%%% Calculated Variables
     view_num_total=view_core(vc)*2^(ringNum(vc) - 1); % Number
     of projections, determined by view_core (vc)*2^(ringNum - 1) =
     view_num_total
     subapertures = view_num_total/view_core(vc); %Number of
     interleaves (Choose a value of 2^n where n is any positive
     integer)
     % if ringNum > 1
     % sino_stack = repmat(s(1:Nproj/2,:),[1 1
     subapertures]) ;
     % t_total = subapertures-1;
     % end
     num_data_sets = (t_total/t_step+1)/subapertures; % How
     many full sets of k-space you want to acquire. Each data set
     should satisfy the nyquist criteria.
     readPoints=2*Nd; %Used for zeropadding
     s_offset=Nd/2-0.25; %central detector offset
     ds=FOV/Nd; %detector spacing (in cm)
     Total_images = ((t_total/t_step+1)/subapertures-
     1)*subapertures + 1; %Total images that can be reconstructed
     % std_n_str = num2str(std_n); %Used for
     writing noise std as a string
     Save_name = ['RSNA_Worspace_Ndetectors' int2str(Nd) '_FOV'
     int2str(ds*Nd) '_vc'...
         int2str(view_core(vc)) '_vnt' int2str(view_num_total)
         '_rn' int2str(ringNum(vc)) ...
          '_SA' int2str(subapertures) '_total_images'
          int2str(Total_images) ...
          '_nyq' int2str(nyq_str(ny))];
``` |
| ```
     theta0_1 = sub_angles_start(subapertures,
     view_core(vc),tube_angles(angle_first)); %Starting angle of
     subapertures
     theta0_2 = repmat(theta0_1, [1
     ceil(time_length/subapertures)]);
     theta0 = theta0_2(1:time_length);
     %%%%%%%%%%%%% kbt tble for Radial recon
     L=4;
     B=pi*L/2;
     kbt=zeros(L/2*100+1,1) ;
     for i=0:L/2*100
         kbt(i+1)=kb(i/100,B,L) ;
     end
     %%%%%%%%%%%%% Initialized variables
     sino_stack_1 = zeros(view_core(vc),Nd,(t_total/t_step+1));
     sino_rn = zeros(Nd,view_core(vc),(t_total/t_step+1)) ;
     sino_pn = zeros(Nd,view_core(vc),(t_total/t_step+1));
     sinoFFTscaled =
     zeros(Nd,view_core(vc),(t_total/t_step+1));
     sinoFFT = zeros(Nd,view_core(vc), (t_total/t_step+1)) ;
     sinofftpad=zeros(readPoints/2,view_core(vc),(t_total/t_step+1));
     shift=zeros(1,view_num_total);
     img_FOV=zeros(floor(readPoints/2),floor(readPoints/2),Total_images
     );
     k_space_data = zeros(readPoints/2, readPoints/2,
     Total_images);
     KWIC_mask=zeros(Nd,view_num_total);
      [row,col,z] = size(k_space_data);
     img_dyn=zeros(row/2,col/2,z);
     sino_angle_KWIC = zeros(view_core(vc), time_length);
``` |
| ```
      %%
     %%%%%%%%%%%%% Determining Angles that relate to each
     projection
     % for aa = 1:time_length
     angle = zeros(view_core(vc),time_length);
     for sa = 1:time_length
         angle(:,sa) =
         ((theta0(sa):pi/view_core(vc):pi*0.9999999+tube_angles(angle_first
         )))';
     end
     % end
      %%
     %%%%%%%%%%%%% Undersampling data set
     for j = 1:size(angle,2)
         for i = 1:size(angle,1)
             sino_stack_1(i,:,j) =
             sino_stack_4(find(abs(sino_Angles_2(:,j) - angle(i,j)) <
             Precision),:,j);
             sino_angle_KWIC(i,j) =
             sino_Angles_2(find(abs(sino_Angles_2(:,j) - angle(i,j)) <
             Precision));
         end
     end
     angle2_2 = reshape(sino_angle_KWIC, 1,
     view_core(vc)*time_length);
     sino_stack_1 = permute(sino_stack_1,[2 1 3]);
      %%
``` |
| ```
     %%%%%%%%%%%%% Changing sinogram to k-space
     for ab = 1:(t_total/t_step+1)
         sinoFFT(:,:,ab) = fft(sino_stack_1(:,:,ab),[],1);
         k=(1:Nd).'; %Allocate column vector of k-space indices
         (Matlab assumes Nk=Ns)
         scaling=exp(-sqrt(-1)*2*pi*s_offset*(k-1)/(Nd));
         scaling=repmat(scaling,1,view_core(vc));
         sinoFFTscaled(:,:,ab)=fftshift(scaling.*sinoFFT(:,:,ab),1); %Scale
         FFT and then shift
         sinofftpad(:, :,ab)=sinoFFTscaled(:, :,ab);
     end
     kdata1 = reshape(sinofftpad, readPoints/2,
     view_core(vc)*(t_total/t_step+1));
      %%
      %%%%%%%%%%%%%%%%%%%%%%%%%%%%%%%%%%%%%%%%%
      %%%%%%%
      %%%%%%%%%%%%%%%%%%%%%%%%%%%%%%%%%%%%%%%%%
      %%%%%%%%%%
     %%%%%%%%%%%%%%%%%%%%%% Reconstructing CT Phantom
     %%%%%%%%%%%%%%%%%%%%%%%%%%%
     %%%%%%%%%%%%%%%%%%%%%%%%%%%%%%%%%%%%%%%%%
     %%%%%%%%%
``` |
| ```
     %%%%%%%%%%%%%%%%%%%%%%%%%%%%%%%%%%%%%%%%%%%%%%%%%%%%%%%%%%%%%%%%
     %%%%%%%%%
     A = 'this is working'
     for imgNum=1:Total_images
     %1:floor(angleViewN/view_core(vc))
         %%%%%%%%%%%%% Determines what projections are being
         used, and the order
         if imgNum == 1
             line = 1:view_num_total;
         else
             line = 1+view_core(vc)*(imgNum-
             1):view_num_total+view_core(vc)*(imgNum-1);
         end
         kdata2 = kdata1(:,line);
         %%%%%%%%%%%%% function rearranges data, does a
         density compensation, and grids to cartesian.
          [kdata_Cart_final,
          KWIC_weighting]=Rad2Cart_voronoi_CT(kdata2,angle2_2(1,line) ...
          ,shift,L,kbt,size(line,2),flag_weighted,view_core(vc),flag_kwic,nL
          ine_ext, ...
             ringNum(vc),nyq(ny)); %resample the data to the
             cartesian space
         KWIC_mask = KWIC_weighting;
         k_space_data(:,:,imgNum) = kdata_Cart_final;
          [w,h,nc]=size(kdata2);
         %%%%%%%%%%%%% Taking FFT and scaling to appropriate
         FOV
``` |
| ```
          [a,b]=meshgrid(1:w);
         A=pi*L*(a-w/2)/w;
         B=pi*L/2;
         cx=sin(sqrt(A.^2-B.^2))./sqrt(A.^2-B.^2);
         cx(:,w)=1;
         A=pi*L*(b-w/2)/w;
         cy=sin(sqrt(A.^2-B.^2))./sqrt(A.^2-B.^2);
         cy(w,:)=1;
         c=cx.*cy;
         img1=0;
         img_fullFOV=0;
         obj1=abs(fftshift(ifft2(fftshift(kdata_Cart_final(:,:))))) ;
         obj1=obj1./c;
         img=obj1(w*1/4+1:w*3/4,w*1/4+1:w*3/4);
         img1=img1+img.^2;
         img_fullFOV=img_fullFOV+obj1.^2;
         img_dyn(:,:,imgNum)=rot90(sqrt(img1));
         img_FOV(:,:,imgNum) = rot90(sqrt(img_fullFOV));
         imgNum
     end
     MAX = max((img_dyn(:)));
     max_fov = max(img_FOV(:));
      %%
     clearvars A a angle2_2 b c cx cy df e img img1 img_dyn
     kdata1 kdata2 kdata_Cart_final line obj1 s_noise scaling shift
     sinoFFT sino_pn sino_rn sinofftpad
     save(Save_name)
 end
 end
``` |
| ```
 function coeff=kb(u,B,L)
 a=B*sqrt(1-(2*u/L)^2) ;
 coeff=besseli(0,a)/L;
 function [kdata_cart,
 xr_1]=Rad2Cart_voronoi_CT(kdata_rad,angle,shift,L,kbt,line_sampled
 ,flag_weighted,view_core,flag_kwic,nLine_ext,num_ring,nyq)
 % kdata_rad = kspace radial data in 2D matrix Readpoints x Views
 used to reconstruct image
 % angle = array with associated angle for each radial view
 % shift = 0's array
 % L = 4 most likely kernel width
 % kbt = kaiser bessel table used for deapodization
 % line_sampled = Number of views used to reconstruct image
 % flag_weighted = 1 true
 % view_core = number of views for central part of KWIC recon
 effective temporal resolution
 % flag_kwic = 1 => true
 % nLine_ext= 1 not sure what impact it has if other value
 % num_ring = number of rings for kwic reconstruction
 [w,h,nc]=size(kdata_rad);
 % w = 2 times number of read out points
 % h = number of views used to reconstruct image
 kdata_cart=zeros(w,w,nc);
 % [angle_sorted,I]=sort(angle+(angle>pi)*(-pi)); %sort the angles
 % kdata_rad=kdata_rad(:,I);
 ratio=view_core; %adjust ratio for golden angle
 Rad_fullSampled=floor(line_sampled/pi/ratio);
``` |
| ```
 xr=zeros (w, h) ;
 yr=zeros (w, h) ;
 ringArray=zeros(line_sampled,1) ;
 % for i=1:num_ring
 % if i==1
 % ringArray(line_sampled/2-
 view_core/2+1:line_sampled/2+view_core/2)=1;
 % elseif i==num_ring
 % ringArray(1:line_sampled/2-view_core/2-(i-
 2)*view_core)=i;
 % ringArray(line_sampled/2+view_core/2+(i-
 2)*view_core+1:end)=i;
 % else
 % ringArray(line_sampled/2-view_core/2-(i-
 1)*view-core+1:line_sampled/2-view-core/2-(i-2)*view-core)=i;
 % ringArray(line_sampled/2+view_core/2+(i-
 2)*view_core+1:line_sampled/2+view_core/2+(i-1)*view_core)=i;
 % end
 % end
 if num_ring > 1
 for i=1:num_ring
     if i==1
         ringArray(line_sampled/2 -
         view_core+1:line_sampled/2)=1; %211 to 240 is 1
     elseif i == 2
     ringArray(line_sampled/2+1:line_sampled/2+view_core)=2; %241 to
     270 is 2
     elseif i==num_ring
         ringArray(1:line_sampled/2-2^(i-3)*view_core)=i; %1 to
         120 is 5
``` |
| ```
         ringArray(line_sampled/2+2^(i-3)*view_core+1:end)=i;
         %361 to 480 is 5
     else
         ringArray(line_sampled/2-2^(i-
         2)*view-core+1:line_sampled/2-2^(i-3)*view_core)=i; %181 to 210 is
         3, 121 to 180 is 4
         ringArray(line_sampled/2+2^(i-
         3)*view_core+1:line_sampled/2+2^(i-2)*view_core)=i; %271 to 300 is
         3, 301 to 360 is 4
     end
 end
 else
 ringArray(:) = 1;
 end
 % if num_ring > 1
 %
 % for i=1:num_ring
 % if i==1
 % ringArray(line_sampled/2-
 view_core+1:line_sampled/2)=1; %211 to 240 is 1
 % elseif i == 2
 %
 ringArray(line_sampled/2+1:line_sampled/2+view_core)=2; %241 to
 270 is 2
 % elseif i==num_ring
 % ringArray(1:line_sampled/2-2^(i-3)*view_core)=i; %1
 to 120 is 5
 % ringArray(line_sampled/2+2^(i-3)*view_core+1:end)=i;
 %361 to 480 is 5
 % else
 % ringArray(line_sampled/2-2^(i-
 2)*view_core+1:line_sampled/2-2^(i-3)*view_core)=i; %181 to 210 is
 3, 121 to 180 is 4
``` |
| ```
 % ringArray(line_sampled/2+2^(i-
 3)*view_core+1:line_sampled/2+2^(i-2)*view_core)=i; %271 to 300 is
 3, 301 to 360 is 4
 % end
 % end
 % else
 % ringArray(:) = 1;
 % end
 for i=1:h
 for j=1:w
     xr(j,i)=(j-1-
     w/2)*sin(angle(i))+w/2+shift(i)*cos(angle(i));
     yr(j,i)=(j-1-w/2)*cos(angle(i))+w/2-
     shift(i)*sin(angle(i));
     if (i<=line_sampled)
         k=i;
     else
         k=ceil((i-line_sampled)/(nLine_ext*2));
         if flag_weighted %true
             if (sqrt(((xr(j,i)-w/2)^2+(yr(j,i)-
             w/2)^2))<=Rad_fullSampled)
                 xr(j,i)=0;
                 yr(j,i)=0;
                 continue;
             end
         end
     end
     if flag_kwic %true if == 1
     % if abs(j-1-w/2)<=(ringArray(k)*2-3)*view_core/pi
     %ranges from
         if abs(j-1-w/2)<=(ringArray(k)*2-3)*view_core/pi*nyq
         %ranges from
             xr (j,i)= 0;
``` |
| ```
             yr(j,i)=0;
         end
     end
 end
 end
 xr_1=xr;
 % yr_1=yr;
 xr=xr(:);
 yr=yr(:);
 kdata_rad=reshape(kdata_rad,w*h,nc);
 tmp=xr.^2+yr.^2;
 loc=find(tmp==0) ;
 xr (loc) = [] ;
 yr(loc)=[] ;
 kdata_rad(loc,:)=[];
 num_points=w*h-size(loc,1);
 %% density compensation
 area=voronoidens_KWIC(xr,yr,w) ;
 area=area(:) ;
 area=repmat(area,[1 nc]);
 %%
 kdata_rad=kdata_rad.*area; %density compensation
 %tic
 for i=1:num_points
 % tic
``` |
| ```
 % percent_done = i/num_points*100;
 %
 % if mod(percent_done,10) == 0
 % disp('Percent completed = ')
 % disp(percent_done)
 % disp('%');
 % end
 xn=floor(xr(i));
 yn=floor(yr(i));
 if (xr(i)<L/2∥xr(i)>(w-1-L/2)∥yr(i)<L/2∥yr(i)>(w-1-
 L/2))
     continue;
 end
 % tic
 for m=xn-L/2+1:xn+L/2
     distx=abs(m-xr(i));
     kbx=kbt(floor(distx*100+0.5)+1);
     for n=yn-L/2+1:yn+L/2
         disty=abs(n-yr(i));
         kby=kbt(floor(disty*100+0.5)+1);
         kdata_cart(m,n,:)=kdata_cart(m,n,:)+reshape(kdata_rad(i,:)*kbx*kby
         ,1,1,nc);
     end
 end
 % toc
 % toc
 end
 %toc
``` |
| ```
 function sub_angles_0 =
 sub_angles_start(subapertures,Nth,angle_first)
 % This function determines the starting angle for each subaperture
 % suberapertues = number of subapertures used for reconstruction
 % Nth = number of angles/projections in the center ring
 % Example Number of subapertures 16
 % theta = [0, 8/16, 4/16, 12/16, 6/16, 14/16, 2/16, 10/16, 5/16,
 13/16, 3/16, 11/16, 1/16, 9/16, 15/16, 7/16]*pi/Nth
 a = zeros(1, subapertures);
 a(1) = 0;
 b = 1;
 c = 2;
 if subapertures >= 2
 for i = 2:subapertures
     if mod(i,2) == 0
         a(i) = a(i-1) + 1/2;
         if a (i) > 1
             a(i) = a(i) - 1;
         end
     elseif mod(c-1,2^b) == 0
         if i > 3
             b = b+1;
         end
         c = 1;
         a(i) = a(i-1)*(1/2);
     else
         a (i) = a(i-1)-(2^b+2)/(2^(b+1)) ;
         if a(i) < 0
``` |
| ```
             a(i) = a(i) + 1;
         end
     end
        c = c + 1;
 end
 end
 sub_angles_0 = pi*a/Nth+angle_first;
 angles_degrees = sub_angles_0/pi*180;
``` |
| ```
 function area=voronoidens_KWIC(kx,ky,w)
 % function area = voronoidens(kx,ky);
 % input: kx, ky are k-space trajectories
 % output: area of cells for each point
 % [row,column] = size(kx);
 tmp=abs(kx-w/2)+abs(ky-w/2) ;
 loc=find(tmp==0) ;
 num_center=size(loc,1) ;
 loc_first=loc(1);
 loc=loc(2:end,1);
 kx(loc)=[] ;
 ky(loc)=[] ;
 kxy = [kx(:),ky(:)];
 % returns vertices and cells of
 % voronoi diagram
 [V,C] = voronoin(kxy,{'QJ','Pp'});
 % unpack cell array, compute
 % area of each ploygon
 area = zeros(length(C),1);
 % for j = 1:length(kxy)
 % x = V(C{j},1);
 % y = V(C{j},2);
 % % lxy = length (x) ;
 % % A = abs(sum(0.5*(x([2:lxy1])-x(:)).*(y([2:lxy
 1])+y(:))));
 % % area = [area A];
 % area_tmp=polyarea(x,y);
``` |
| ```
 % end
 for j = 1:length(kxy)
 if all(C{j}∼=1)
     x = V(C{j},1) ;
     y = V(C{j},2);
     % if (all(x<=w) && all(x>=0) && all(y<=w) && all(y>=0))
     if (all(sqrt((x-w/2).^2+(y-w/2).^2)<w/2))
         area(j)=polyarea(x,y) ;
     else
         area(j)=0;
     end
 else
     area(j)=0;
 end
 end
 area(loc_first)=area(loc_first)/num_center;
 for i=1:length(loc)
 area=[area(1:loc(i)-1); area(loc_first); area(loc(i):end)];
 end
 % area = reshape(area,row,column);
``` |

## Claims

1. A system provided for operating with a CT scanner comprising an X-ray source mounted on gantry so as to rotate within a cylindrical enclosure of the CT scanner, the system comprising:
(a) a pulse generator configured to be coupled to the X-ray source;
the pulse generator configured to periodically switch off emission of X-rays from the X-ray source into the cylindrical enclosure;
(b) application software coupled to the pulse generator;
the application software comprising instructions to control timing of the pulse generator so as to intermittently expose a subject to X-rays from the X-ray source at pre-specified rotation angles of the gantry;
(c) application software coupled to an output of the CT scanner for receiving pulsed images from the CT scanner, the pulsed images corresponding to exposures at said pre-specified rotation angles;
the application software comprising instructions for reconstructing each of said exposures to generate a reconstructed image; and
(d) wherein weighted image contrast is used to generate the reconstructed image via projection view sharing, wherein a central 2D Fourier Transform (2DFT) space, which determines the image contrast, is sampled by projection views of a time frame of interest, and wherein a peripheral 2DFT space is filled by projection views of time frames neighboring the time frame of interest.

2. The system of claim 1:
wherein the pulse generator comprises a mechanical shutter coupled to the x-ray source;
wherein the shutter comprises an off-state to restrict X-rays from being emitted from the X-ray source and an on-state configured to allow X-rays to be emitted from the X-ray source into the enclosure;
wherein the shutter is coupled to the application software to receive said instructions; and
wherein the instructions comprise commands for timing the on-state of the shutter and resulting X-ray exposure at said pre-specified rotation angles of the gantry.

3. The system of claim 1:
wherein the pulse generator comprises an off-state to restrict X-rays from being emitted from the X-ray pulse generator source and an on-state configured to allow X-rays to be emitted from the X-ray source into the enclosure;
wherein the pulse generator is coupled to the X-ray source to electromagnetically shield the X-ray source from emitting X-rays in the off-state; and
wherein the instructions comprise commands for timing the on-state of the pulse generator and resulting X-ray exposure at said pre-specified rotation angles of the gantry.

4. The system of claim 3:
wherein the X-ray source comprises an anode, a cathode and a griddling electrode there between; and
wherein the pulse generator is configured to modify a negative potential of the griddling electrode to form an electromagnetic field-based shield so as to prevent electron flow from the cathode the anode, thereby stopping emission of X-rays in the off-state.

5. The system of claim 1, wherein said pre-specified angles of rotation comprise a sequence of rotation angles selected from the group of rotation schemes consisting of: an angle-bisect scheme; a Golden-ratio scheme; or a Tiny Golden-ratio scheme.

6. The system of claim 1, further comprising:
(a) a computer processor coupled to the CT scanner; and
(b) a non-transitory computer-readable memory storing said instructions;
(c) wherein said instructions, when executed by the computer processor, further perform the steps for reconstructing each of said exposures to generate the reconstructed image.

7. A method of imaging a subject a CT scanner, the CT scanner comprising an X-ray source mounted on gantry so as to rotate within a cylindrical enclosure of the CT scanner and emit of X-rays into the cylindrical enclosure, the method comprising:
(a) intermittently exposing a subject within the enclosure to X-rays from the X-ray source at pre-specified rotation angles of the gantry;
(b) receiving pulsed images from the CT scanner, the pulsed images corresponding to exposures at said pre-specified rotation angles; and
(c) reconstructing each of said exposures to generate a reconstructed image;
(d) wherein weighted image contrast is used to generate the reconstructed image via projection view sharing, wherein a central 2D Fourier Transform (2DFT) space, which determines the image contrast, is sampled by projection views of a time frame of interest, and wherein a peripheral 2DFT space is filled by projection views of time frames neighboring the time frame of interest.

8. The method of claim 7: wherein the CT scanner comprises a mechanical shutter coupled to the x-ray source;
wherein the shutter comprises an off-state to restrict X-rays from being emitted from the X-ray source and an on-state configured to allow X-rays to be emitted from the X-ray source into the enclosure; and
wherein intermittently exposing a subject comprises timing the on-state of the shutter and resulting X-ray exposure at said pre-specified rotation angles of the gantry.

9. The method of claim 7:
wherein the X-ray source comprises an electron beam being focused on an anode from a cathode to generate said X-rays; and
wherein intermittently exposing a subject comprises deflecting the electron beam off the anode using a magnetic field, thereby restricting emission of X-rays from the X-ray source into the enclosure to control X-ray exposure to the subject only at said pre-specified rotation angles of the gantry.

10. The method of claim 9: the X-ray source further comprising:
a griddling electrode between the anode and the cathode;
wherein deflecting the electron beam off the anode comprises generating sufficient negative potential within the griddling electrode to form an electromagnetic field-based shield so as to prevent electron flow from the cathode the anode, thereby stopping emission of X-rays for pre-specified rotation angles.

11. The method of claim 7, wherein said pre-specified angles of rotation comprise a sequence of rotation angles selected from the group of rotation schemes consisting of: an angle-bisect scheme; a Golden-ratio scheme; or a Tiny Golden-ratio scheme.

## Patentansprüche

1. System, das für den Betrieb mit einem CT-Scanner bereitgestellt ist, der eine Röntgenstrahlquelle umfasst, die an einem Gestell angebracht ist, so dass sie sich in einer zylindrischen Einfassung des CT-Scanners dreht, wobei das System folgendes umfasst:
(a) einen Impulsgenerator, der für eine Kopplung mit der Röntgenstrahlquelle gestaltet ist;
wobei der Impulsgenerator so gestaltet ist, dass er die Emission von Röntgenstrahlen von der Röntgenstrahlquelle in die zylindrische Einfassung periodisch abschaltet;
(b) Anwendungssoftware, die mit dem Impulsgenerator gekoppelt ist;
wobei die Anwendungssoftware Anweisungen zur Steuerung der Zeitsteuerung des Impulsgenerators umfasst, um ein Subjekt periodisch Röntgenstrahlen von der Röntgenstrahlquelle in vorab festgelegten Rotationswinkeln auszusetzen;
(c) Anwendungssoftware, die mit einem Ausgang des CT-Scanners gekoppelt ist, um gepulste Bilder von dem CT-Scanner zu empfangen, wobei die gepulsten Bilder Expositionen bei den vorab festgelegten Rotationswinkeln entsprechen;
wobei die Anwendungssoftware Anweisungen zur Rekonstruktion jeder der Expositionen umfasst, um ein rekonstruiertes Bild zu erzeugen; und
(d) wobei ein gewichteter Bildkontrast verwendet wird, um das rekonstruierte Bild über Teilen der Projektionsansicht zu erzeugen, wobei ein zentraler Raum einer 2D Fourier-Transformation (2DF), der den Bildkontrast bestimmt, durch Projektionsansichten eines relevanten Zeitrahmens abgetastet wird, und wobei ein peripherer 2DFT-Raum mit Projektionsansichten von Zeitrahmen gefüllt ist, die zu dem relevanten Zeitrahmen benachbart sind.

2. System nach Anspruch 1,
wobei der Impulsgenerator einen mechanischen Verschluss umfasst, der mit der Röntgenstrahlquell gekoppelt ist;
wobei der Verschluss einen ausgeschalteten Zustand umfasst, um zu verhindern, dass Röntgenstrahlen von der Röntgenstrahlquelle emittiert werden, und einen eingeschalteten Zustand, der so gestaltet ist, dass er die Emission von Röntgenstrahlen von der Röntgenstrahlquelle in die Einfassung zulässt;
wobei der Verschluss mit der Anwendungssoftware gekoppelt ist, um die Anweisungen zu empfangen; und
wobei die Anweisungen Befehle zur Zeitsteuerung des eingeschalteten Zustands des Verschlusses umfassen und der folgenden Röntgenstrahlexposition bei den vorab festgelegten Rotationswinkeln des Gestells.

3. System nach Anspruch 1,
wobei der Impulsgenerator einen ausgeschalteten Zustand umfasst, um zu verhindern, dass Röntgenstrahlen von der Röntgenstrahlimpulsgeneratorquelle emittiert werden, und einen eingeschalteten Zustand, der so gestaltet ist, dass er die Emission von Röntgenstrahlen von der Röntgenstrahlquelle in die Einfassung zulässt;
wobei der Impulsgenerator mit der Röntgenstrahlquelle gekoppelt ist, um die Röntgenstrahlquelle elektromagnetisch gegen die Emission von Röntgenstrahlen in dem ausgeschalteten Zustand abzuschirmen; und
wobei die Anweisungen Befehle zur Zeitsteuerung des eingeschalteten Zustands des umfassen und der folgenden Röntgenstrahlexposition bei den vorab festgelegten Rotationswinkeln des Gestells.

4. System nach Anspruch 3,
wobei die Röntgenstrahlquelle eine Anode, eine Kathode und eine Grillelektrode dazwischen umfasst; und
wobei der Impulsgenerator so gestaltet ist, dass er ein negatives Potential der Grillelektrode modifiziert, um eine auf einem elektromagnetischen Feld basierende Abschirmung zu bilden, um einen Elektronenfluss von er Kathode zu der Anode zu verhindern, um dadurch die Emission von Röntgenstrahlen in dem ausgeschalteten Zustand zu beenden.

5. System nach Anspruch 1, wobei die vorab festgelegten Rotationswinkel eine Folge von Rotationswinkeln umfassen, die ausgewählt sind aus Rotationsmustern, die folgendes umfassen: ein Winkelhalbierendenmuster; ein Goldener-Schnitt-Muster, oder ein keines Goldener-Schnitt-Muster.

6. System nach Anspruch 1, wobei dieses ferner folgendes umfasst:
(a) einen Computerprozessor, der mit dem CT-Scanner gekoppelt ist; und
(b) einen nichtflüchtigen, computerlesbaren Speicher, der die Anweisungen speichert;
(c) wobei die Anweisungen, wenn sie durch den Computerprozessor ausgeführt werden, ferner die Schritte der Rekonstruktion jeder der Expositionen ausführen, um das rekonstruierte Bild zu erzeugen.

7. Verfahren zur bildlichen Darstellung eines Subjekts in einem CT-Scanner, wobei der CT-Scanner eine an einem Gestell montierte Röntgenstrahlquelle umfasst, so dass sich diese in einer zylindrischen Einfassung des CT-Scanners dreht und Röntgenstrahlen in die zylindrische Einfassung emittiert, wobei das Verfahren folgendes umfasst:
(a) periodisches Aussetzen eines Subjekts in der Einfassung Röntgenstrahlen von der Röntgenstrahlquelle in den vorab festgelegten Rotationswinkeln des Gestells;
(b) Empfangen gepulster Bilder von dem CT-Scanner, wobei die gepulsten Bilder Expositionen bei den vorab festgelegten Rotationswinkeln entsprechen; und
(c) Rekonstruieren jeder der Expositionen, um ein rekonstruiertes Bild zu erzeugen;
(d) wobei ein gewichteter Bildkontrast verwendet wird, um das rekonstruierte Bild über Teilen der Projektionsansicht zu erzeugen, wobei ein zentraler Raum einer 2D Fourier-Transformation (2DF), der den Bildkontrast bestimmt, durch Projektionsansichten eines relevanten Zeitrahmens abgetastet wird, und wobei ein peripherer 2DFT-Raum mit Projektionsansichten von Zeitrahmen gefüllt ist, die zu dem relevanten Zeitrahmen benachbart sind.

8. Verfahren nach Anspruch 7, wobei der CT-Scanner einen mechanischen Verschluss umfasst, der mit der Röntgenstrahlquelle gekoppelt ist;
wobei der Verschluss einen ausgeschalteten Zustand umfasst, um zu verhindern, dass Röntgenstrahlen von der Röntgenstrahlquelle emittiert werden, und einen eingeschalteten Zustand, der so gestaltet ist, dass er die Emission von Röntgenstrahlen von der Röntgenstrahlquelle in die Einfassung zulässt; und
wobei das periodische Aussetzen eines Subjekts die Zeitsteuerung des eingeschalteten Zustands des Verschlusses umfasst und der folgenden Röntgenstrahlexposition bei den vorab festgelegten Rotationswinkeln des Gestells.

9. Verfahren nach Anspruch 7,
wobei die Röntgenstrahlquelle einen Elektronenstrahl umfasst, der von einer Kathode auf eine Anode fokussiert ist, um die Röntgenstrahlen zu erzeugen; und
wobei das periodische Aussetzen eines Subjekts das Ablenken des Elektronenstrahls von der Anode unter Verwendung eines Magnetfelds umfasst, wodurch die Emission von Röntgenstrahlen von der Röntgenstrahlquelle in die Einfassung eingeschränkt wird, um die Röntgenstrahlexposition des Subjekts nur mit den spezifizierten Rotationswinkeln des Gestells zu steuern.

10. Verfahren nach Anspruch 9, wobei die Röntgenstrahlquelle ferner folgendes umfasst:
eine Grillelektrode zwischen der Anode und der Kathode;
wobei das Ablenken des Elektronenstrahls von der Anode das Erzeugen von ausreichend negativem Potential in der Grillelektrode umfasst, um eine Abschirmung auf der Basis eines elektromagnetischen Felds zu bilden, um einen Elektronenfluss von der Kathode zu der Anode zu verhindern, wodurch die Emission von Röntgenstrahlen für vorab festgelegte Rotationswinkel beendet wird.

11. Verfahren nach Anspruch 7, wobei die vorab festgelegten Rotationswinkel eine Folge von Rotationswinkeln umfassen, die ausgewählt sind aus Rotationsmustern, die folgendes umfassen: ein Winkelhalbierendenmuster; ein Goldener-Schnitt-Muster, oder ein keines Goldener-Schnitt-Muster.

## Revendications

1. Système destiné à fonctionner avec un scanner CT comprenant une source de rayons X montée sur un portique de sorte à tourner à l'intérieur d'une enceinte cylindrique du scanner CT, le système comprenant :
(a) un générateur d'impulsions conçu pour être couplé à la source de rayons X ;
le générateur d'impulsions étant conçu pour arrêter périodiquement l'émission de rayons X de la source de rayons X dans l'enceinte cylindrique ;
(b) un logiciel d'application couplé au générateur d'impulsions ;
le logiciel d'application comprenant des instructions pour commander la synchronisation du générateur d'impulsions de sorte à exposer par intermittence un sujet aux rayons X de la source de rayons X à des angles de rotation prédéfinis du portique ;
(c) un logiciel d'application couplé à une sortie du scanner CT pour recevoir des images pulsées du scanner CT, les images pulsées correspondant à des expositions auxdits angles de rotation prédéfinis ;
le logiciel d'application comprenant des instructions pour reconstruire chacune desdites expositions afin de générer une image reconstruite ; et
(d) un contraste d'image pondéré étant utilisé pour générer l'image reconstruite par l'intermédiaire d'un partage de vue de projection, un espace central de transformation de Fourier 2D (2DFT), qui détermine le contraste d'image, étant échantillonné par des vues de projection d'une période de temps d'intérêt, et un espace 2DFT périphérique étant rempli par des vues de projection de périodes de temps voisines de la période de temps d'intérêt.

2. Système selon la revendication 1 :
le générateur d'impulsions comprenant un obturateur mécanique couplé à la source de rayons X ;
l'obturateur comprenant un état d'arrêt pour empêcher les rayons X d'être émis par la source de rayons X et un état de marche conçu pour permettre aux rayons X d'être émis par la source de rayons X dans l'enceinte ;
l'obturateur étant couplé au logiciel d'application pour recevoir lesdites instructions ; et
les instructions comprenant des commandes pour synchroniser l'état de marche de l'obturateur et l'exposition aux rayons X qui en résulte auxdits angles de rotation prédéfinis du portique.

3. Système selon la revendication 1 :
le générateur d'impulsions comprenant un état d'arrêt pour empêcher les rayons X d'être émis par la source de générateur d'impulsions de rayons X et un état de marche conçu pour permettre aux rayons X d'être émis par la source de rayons X dans l'enceinte ;
le générateur d'impulsions étant couplé à la source de rayons X pour protéger électromagnétiquement la source de rayons X contre l'émission de rayons X à l'état d'arrêt ; et
les instructions comprenant des commandes pour synchroniser l'état de marche de générateur d'impulsions et l'exposition aux rayons X qui en résulte auxdits angles de rotation prédéfinis du portique.

4. Système selon la revendication 3 :
la source de rayons X comprenant une anode, une cathode et une électrode de grille entre les elles ; et
le générateur d'impulsions étant conçu pour modifier un potentiel négatif de l'électrode de grille afin de former un écran basé sur un champ électromagnétique de sorte à empêcher le flux d'électrons de la cathode vers l'anode, arrêtant ainsi l'émission de rayons X à l'état de repos.

5. Système selon la revendication 1, lesdits angles de rotation prédéfinis comprenant une séquence d'angles de rotation sélectionnés dans le groupe de schémas de rotation comprenant : un schéma angle-bissectrice ; un schéma de nombre d'or ; ou un schéma de nombre d'or étroit.

6. Système selon la revendication 1, comprenant en outre :
(a) un processeur informatique couplé au scanner CT ; et
(b) une mémoire non transitoire lisible par ordinateur stockant lesdites instructions ;
(c) lesdites instructions, lorsqu'elles sont exécutées par le processeur de l'ordinateur, exécutant en outre les étapes de reconstruction de chacune desdites expositions pour générer l'image reconstruite.

7. Procédé d'imagerie d'un sujet dans un scanner CT, le scanner CT comprenant une source de rayons X montée sur un portique de sorte à tourner à l'intérieur d'une enceinte cylindrique du scanner CT et à émettre des rayons X dans l'enceinte cylindrique, le procédé comprenant les étapes consistant à :
(a) exposer par intermittence un sujet se trouvant dans l'enceinte aux rayons X de la source de rayons X à des angles de rotation prédéfinis du portique ;
(b) recevoir des images pulsées du scanner CT, les images pulsées correspondant aux expositions auxdits angles de rotation prédéfinis ; et
(c) reconstruire chacune desdites expositions pour générer une image reconstruite ;
(d) un contraste d'image pondéré étant utilisé pour générer l'image reconstruite par l'intermédiaire d'un partage de vue de projection, un espace central de transformation de Fourier 2D (2DFT), qui détermine le contraste d'image, étant échantillonné par des vues de projection d'une période de temps d'intérêt, et un espace 2DFT périphérique étant rempli par des vues de projection de périodes de temps voisines de la période de temps d'intérêt.

8. Procédé selon la revendication 7 : le scanner CT comprenant un obturateur mécanique couplé à la source de rayons X ;
l'obturateur comprenant un état d'arrêt pour empêcher les rayons X d'être émis par la source de rayons X et un état de marche conçu pour permettre aux rayons X d'être émis par la source de rayons X dans l'enceinte ; et
l'exposition intermittente d'un sujet comprenant la synchronisation de l'état de marche de l'obturateur et l'exposition aux rayons X résultante auxdits angles de rotation pré-spécifiés du portique.

9. Procédé selon la revendication 7 :
la source de rayons X comprenant un faisceau d'électrons qui est focalisé sur une anode à partir d'une cathode pour générer lesdits rayons X ; et
l'exposition intermittente d'un sujet comprenant l'étape consistant à dévier le faisceau d'électrons hors de l'anode en utilisant un champ magnétique, limitant ainsi l'émission de rayons X de la source de rayons X dans l'enceinte pour commander l'exposition aux rayons X du sujet uniquement auxdits angles de rotation pré-spécifiés du portique.

10. Procédé selon la revendication 9 : la source de rayons X comprenant en outre :
une électrode de grille entre l'anode et la cathode ;
la déviation du faisceau d'électrons hors de l'anode comprenant l'étape consistant à générer un potentiel négatif suffisant au sein de l'électrode de grille pour former un écran basé sur un champ électromagnétique de sorte à empêcher le flux d'électrons de la cathode vers l'anode, arrêtant ainsi l'émission de rayons X pour des angles de rotation prédéfinis.

11. Procédé selon la revendication 7, lesdits angles de rotation prédéfinis comprenant une séquence d'angles de rotation sélectionnés dans le groupe de schémas de rotation comprenant : un schéma angle-bissectrice ; un schéma de nombre d'or ; ou un schéma de nombre d'or étroit.
